(19) Europäisches Patentamt
European Patent Office
Office européen des brevets

(11) **EP 3 872 087 B1**

(12) **EUROPEAN PATENT SPECIFICATION**

(45) Date of publication and mention of the grant of the patent:
**25.09.2024 Bulletin 2024/39**

(21) Application number: **19877316.0**

(22) Date of filing: **25.10.2019**

(51) International Patent Classification (IPC):
**C07K 14/705** (2006.01)   **C12N 5/0783** (2010.01)
**C12N 5/10** (2006.01)   **C12N 15/12** (2006.01)
**C12N 15/63** (2006.01)

(52) Cooperative Patent Classification (CPC):
**C07K 14/705; A61K 39/4611; A61K 39/4631;**
**A61K 39/464402; C07K 14/7051; C12N 5/0636;**
**C12N 15/85;** A61K 48/00; A61K 2239/17;
C07K 14/535; C07K 14/5406; C12N 2501/22;
C12N 2501/2304; C12N 2501/2307;
C12N 2501/2315;   (Cont.)

(86) International application number:
**PCT/JP2019/041878**

(87) International publication number:
**WO 2020/085480 (30.04.2020 Gazette 2020/18)**

(54) **HIGH-EFFICIENCY METHOD FOR PRODUCING GENETICALLY MODIFIED CELLS**

HOCHEFFIZIENTES VERFAHREN ZUR HERSTELLUNG GENETISCH MODIFIZIERTER ZELLEN

PROCÉDÉ À HAUT RENDEMENT POUR LA PRODUCTION DE CELLULES GÉNÉTIQUEMENT MODIFIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 JP 2018202336**

(43) Date of publication of application:
**01.09.2021 Bulletin 2021/35**

(73) Proprietor: **Shinshu University Matsumoto-shi, Nagano 390-8621 (JP)**

(72) Inventors:
• **NAKAZAWA, Yozo** Nagano 390-8621 (JP)
• **TANAKA, Miyuki** Nagano 390-8621 (JP)
• **MOROKAWA, Hirokazu** Nagano 390-8621 (JP)
• **NARIMATSU, Shogo** Nagano 399-8304 (JP)

(74) Representative: **Vossius & Partner Patentanwälte Rechtsanwälte mbB Siebertstraße 3 81675 München (DE)**

(56) References cited:
**WO-A1-2017/061615   WO-A1-2017/079705**
**WO-A1-2020/014366   WO-A1-2021/019706**
**JP-A- 2013 529 061   JP-A- 2018 531 014**

• **KEBRIAEI PARTOW ET AL: "Adoptive Therapy Using Sleeping Beauty Gene Transfer System and Artificial Antigen Presenting Cells to Manufacture T Cells Expressing CD19-Specific Chimeric Antigen Receptor", BLOOD, AMERICAN SOCIETY OF HEMATOLOGY, US, vol. 124, no. 21, 14 November 2014 (2014-11-14), pages 311, XP086740173, ISSN: 0006-4971, [retrieved on 20210625], DOI: 10.1182/BLOOD.V124.21.311.311**

Remarks:
  •The complete document including Reference Table(s) and the Sequence Listing(s) can be downloaded from the EPO website
  •The file contains technical information submitted after the application was filed and not included in this specification

(52) Cooperative Patent Classification (CPC): (Cont.)
C12N 2510/00; C12N 2800/90

**Description**

Technical Field

The present invention relates to a method for producing genetically modified cells expressing a chimeric antigen receptor, which is useful in the field of adoptive immunotherapy.

Background Art

Adoptive immunotherapy using T cells allowed to express a chimeric antigen receptor (CAR) (CAR-T) targeting a tumor-related antigen reportedly has a potent antitumor effect, and its development has advanced rapidly in recent years. Particularly, the development of CAR aimed at the treatment of B cell tumor has advanced, already reaching clinical application (Non Patent Literatures 1 and 2). During research and development on the CAR-T technology, the importance of the step of producing CAR-T cells on a clinical grade has been recognized for the clinical application of CAR-T cells serving as cell medicaments.

Heretofore, the CAR-T cells have been produced using virus vectors. However, problems associated with safety or facilities have been pointed out as to such production methods using virus vectors (Non Patent Literatures 3 and 4). In order to solve the problems of the conventional CAR therapy using virus vectors, use of a transposon method, one of the genetic modification techniques using non-viral vectors, has been studied. On the other hand, the transposon method has low gene introduction efficiency as compared with the virus vector methods, and often requires the step of selecting CAR-expressing cells and/or a long culture period for clinical application (Non Patent Literature 5). Furthermore, problems have been pointed out, such as decrease in cell survival rate or cell growth rate caused by the damage of cells in association with gene introduction by electroporation. Since the clinical application of T cell treatment requires T cells on the order of at least $1 \times 10^6$ cells/kg in terms of the number of CAR-positive cells, a low expression rate of CAR-T cells makes the clinical application difficult (Non Patent Literature 6).

In order to solve these problems, various studies aimed at increase in the number of CAR-expressing cells have been made for the step of producing CAR-T cells by use of the transposon method.

Conventional viral peptide methods exploit, for example, a rapid expansion protocol (REP) using irradiated feeder cells derived from peripheral blood mononuclear cells (PBMCs), and an anti-CD3 antibody (Patent Literature 1), or an approach of nonspecifically stimulating T cells by immobilizing an anti-CD3 antibody or an anti-CD28 antibody onto accessory cells, beads or solid surface (Non Patent Literatures 4 and 7).

For the preparation of CD19.CAR-T cells using piggyBac, one of the transposons, an approach of activating CAR-T cells in a CAR-specific manner has been developed, in addition to conventional methods, which forces a tumor cell line such as K562 to express CD19 or a costimulatory factor to separately prepare artificial antigen presenting cells (aAPCs), and coculturing these cells with cells harboring introduced CAR gene (Non Patent Literature 8). Also, methods of coculturing cells harboring introduced CAR gene with cells obtained by activating irradiated peripheral blood mononuclear cells (PBMCs) using an anti-CD3 antibody or an anti-CD28 antibody (ATCs method) or by further irradiating with viral peptide pulse (ACE method) as feeder cells have been developed and found effective (Patent Literature 2 and Non Patent Literature 9). Previously, an adoptive therapy using a sleeping beauty gene transfer system and artificial antigen presenting cells to manufacture T cells expressing CD19-specific chimeric antigen receptor has been described (Partow et al., Blood, American Society of Hematology Vol.1 No. 21:311, 2014.

However, these methods still require complicated steps. Furthermore, additional complicated steps are generated for the preparation of aAPCs using a tumor cell line or the preparation of feeder cells using a viral peptide. Moreover, these approaches might cause complicated problems associated with regulation toward clinical application. In addition, there arise problems associated with cost due to use of a plurality of components such as an anti-CD3 antibody and an anti-CD28 antibody. Hence, for the transposon method, it is desirable to develop a production method that effectively contributes to increase in the number of CAR-expressing cells using more convenient steps.

Citation List

Patent Literature

Patent Literature 1: U.S. Patent No. 5,827,642
Patent Literature 2: WO2017/061615

Non Patent Literature

Non Patent Literature 1: N. Engl. J. Med. (2017), 377, 2531-2544
Non Patent Literature 2: N. Engl. J. Med. (2018), 378, 439-448
Non Patent Literature 3: J Immunotherapy (2013), 36 (1), 1-2
Non Patent Literature 4: Current Opinion in Biotechnology (2018) 53: 164-181
Non Patent Literature 5: Cytotherapy (2014) 16, 1257-1269
Non Patent Literature 6: Lancet (2015), 385, 517-528
Non Patent Literature 7: J Immunol Methods (2003) Apr 1; 275 (1-2): 251-5
Non Patent Literature 8: Human Gene Therapy (2010) 21: 427-437
Non Patent Literature 9: Molecular Therapy: Methods & Clinical Development (2018) 3: 131-140

Summary of Invention

Technical Problem

As described above, there is demand for a method for producing CAR-T cells, which achieves increase in the number of CAR-expressing cells conveniently and effectively by use of a transposon method.

Solution to Problem

The present inventors have conducted diligent studies to solve the problems described above. As a result, the present inventors have found a method for producing CAR-T cells, which achieves increase in the number of CAR-expressing cells using much more convenient steps than those of conventional production methods, through the use of cells expressing a substance that binds to CAR contained in human primary cultured cells.
Specifically, the present invention relates to the embodiments as characterized in the claims.
Thus, it relates to the following items:

[1] A method for producing genetically modified mammalian cells, comprising the steps of:

a) introducing a polynucleotide encoding a chimeric antigen receptor (CAR) protein to a cell population comprising T cells derived from a mammal by a transposon method to obtain a genetically modified cell population;
b) providing a cell population derived from PBMCs derived from the mammal expressing a protein that binds to the CAR, wherein said cell population is cultured in the presence of one or more cytokines, wherein said cytokine is GM-CSF and/or IL-4 and wherein said derived PBMCs are PBMCs used without inactivation treatment; and
c) coculturing the genetically modified cell population of the step a) and the cell population of the step b).

[2] The method according to item 1, wherein the step c) is performed in the presence of one or more cytokines.
[3] The method according to item 2, wherein the cytokine in the step c) is IL-7 and/or IL-15.
[4] The method according to any one of items 1 to 3, wherein the transposon method is a piggyBac method.
[5] The method according to any one of items 1 to 4, wherein the genetically modified cell population and the cell population are derived from the same individual.
[6] The method according to any one of items 1 to 5, wherein the CAR protein is a protein comprising a binding domain specific for human granulocyte-macrophage colony-stimulating factor (GM-CSF) receptor, and the endogenous cell population is GM-CSF receptor-expressing cells.

Advantageous Effects of Invention

The method for producing genetically modified cells according to the present invention is capable of increasing the number of CAR-expressing cells using much more convenient steps than those of conventional production methods. CAR-T cells produced by this method exhibit equivalent or higher efficacy as compared with the conventional production methods.

Brief Description of Drawings

[Figure 1] Figure 1 shows a vector map of CAR001.
[Figure 2] Figure 2 shows an exemplary vector map of CAR002 having a modified spacer domain (CH2CH3 partial deletion-(G4S)3 insertion mutant, GMR.CAR dCH2CH3+(G4S)3).
[Figure 3] Figure 3 shows an exemplary vector map of CAR003 having a modified spacer domain (CH2CH3 partial deletion mutant, E21KGMR.CAR dCH2CH3).
[Figure 4] Figure 4 shows an exemplary vector map of CAR004 having a modified spacer domain (CH2CH3 partial deletion mutant, E21KGMR.CAR dCH2CH3).
[Figure 5] Figure 5 shows expression analysis results of CAR-T 004 on Day 14.
[Figure 6] Figure 6 shows expression analysis results of CAR-T 004-A on Day 13.
[Figure 7] Figure 7 shows expression analysis results of CAR-T 004-B on Day 10.
[Figure 8] Figure 8 shows results about the evaluation of the antitumor cell activity of CAR-T 004-B against THP-1 cells on Day 4 (d4), Day 8 (d8), Day 12 (d12), Day 15 (d15), Day 19 (d19), and Day 23 (d23).
[Figure 9] Figure 9 shows expression analysis results of CAR-T 004-C on Day 11.
[Figure 10] Figure 10 shows a PD-1-positive rate (exhaustion rate) of CAR-T 004-A and CAR-T 004-B on Day 14.
[Figure 11] Figure 11 shows the proportion of a stem cell memory fraction of CAR-T 004-A and CAR-T 004-B on Day 14.

Description of Embodiments

Hereinafter, some embodiments of the present invention will be described in detail. It should be understood that the term "cell population" is used in the present specification for clearly indicating that a plurality of cells or a plurality of types of cells are included, and may be used interchangeably with "cells", depending on the context.

The present invention provides a method for producing genetically modified cells, comprising the steps of:

a) introducing a polynucleotide encoding a chimeric antigen receptor (CAR) protein to a cell population comprising T cells derived from a mammal by a transposon method to obtain a genetically modified cell population;
b) providing a cell population derived from PBMCs derived from the mammal expressing a protein that binds to the CAR, wherein said cell population is cultured in the presence of one or more cytokines, wherein said cytokine is GM-CSF and/or IL-4 and wherein said derived PBMCs are PBMCs used without inactivation treatment; and
c) coculturing the genetically modified cell population of the step a) and the cell population of the step b).

The method of the present invention can be applied to a mammal and can be suitably carried out for a mammal, for example, a human, a monkey, a mouse, a rat, a pig, cattle, or a dog. The method of the present invention is a method comprising the steps of: introducing a polynucleotide *in vitro* to cells obtained from a mammal; and culturing the cells, and is an *ex vivo* method intended to be used for administering the obtained genetically modified cells to the mammal for adoptive immunotherapy.

[Step a)]

For example, a cell population collected, isolated, purified or induced from a body fluid such as blood (peripheral blood, umbilical cord blood, etc.) or bone marrow, a tissue or an organ can be used as the "cell population comprising T cells" according to the present invention. Peripheral blood mononuclear cells (PBMCs) can be suitably used.

In a preferred embodiment of the present invention, for example, cells releasing a cytotoxic protein (perforin, granzyme, etc.) can be used. Specifically, for example, a cell population containing T cells, precursor cells of T cells (hematopoietic stem cells, lymphocyte precursor cells, etc.), or NK-T cells can be used. Further, cells capable of differentiating into these cells include various stem cells such as ES cells and iPS cells. The T cells include CD8-positive T cells, CD4-positive T cells, regulatory T cells, cytotoxic T cells or tumor-infiltrating lymphocytes. The cell population containing T cells and precursor cells of T cells includes PBMCs. The cells described above may be collected from a living organism, obtained by the expansion culture of the cells, or established as a cell line. In the case of transplanting the produced cells expressing CAR or cells differentiated from the cells into a living organism, it is desirable to introduce a nucleic acid into cells collected from the living organism itself or a living organism of the same species thereas.

T cells expected to have a sustained antitumor effect, for example, stem cell memory T cells, can be used as T cells for use in adoptive immunotherapy to which the polynucleotide is to be introduced to produce the genetically modified cells of the present invention. The stem cell memory T cells can be analyzed according to a routine method and easily confirmed as described in, for example, Yang Xu, et al., Blood. 2014; 123: 3750-3759.

CD45R0-, CD62L+, CD45RA+ and CCR7+ cells, for example, can be used as cells to which the polynucleotide described above is to be introduced. More preferably, a cell population containing at least 20% or more of CD45RA+ and CCR7+ cells can be used as cells to which the polynucleotide described above is to be introduced.

In the present specification, the "chimeric antigen receptor (CAR)" refers to a modified receptor that can impart its target specificity to cells such as T cells (e.g., T cells such as naive T cells, stem cell memory T cells, central memory T cells, effector memory T cells or a combination thereof). CAR is also known as an artificial T cell receptor, a chimeric T cell receptor or a chimeric immunoreceptor.

The CAR for use in the method of the present invention has a target binding domain that specifically binds to a target molecule, a transmembrane domain, and an intracellular signal transduction domain. In this context, the "domain" refers to a region that is within a polypeptide and is folded into a particular structure, independently of other regions.

The method of the present invention can be applied to the general preparation of genetically modified cells expressing CAR for use in adoptive immunotherapy. Thus, the molecule targeted by CAR is typically an antigen found to be significantly or markedly expressed in tumor cells compared with other cells. Examples thereof include, but are not limited to, CD19, CD20, GD2, CD22, CD30, CD33, CD44, CEA, Her2/neu, MUC1, MUC4, MUC6, EGFR, VEGFR2, GM-CSFR, IL-11R$\alpha$, and IL-13$\alpha$2.

The target binding domain of the CAR protein can comprise, for example, a single-chain antibody fragment (scFv) comprising heavy chain (H chain) and light chain (L chain) variable regions of a monoclonal antibody that specifically binds to the target molecule as described above. Those skilled in the art can readily produce a monoclonal antibody against a particular antigen and a scFv fragment that can be used in CAR. Alternatively, commercially available products may be used. Examples of the target binding domain that may be used in this embodiment can include a scFv fragment that specifically binds to CD 19.

Alternatively, when the targeted antigen is a receptor expressed on tumor cell surface, the structure of a ligand that specifically binds to the receptor can be used as the target binding domain. Examples of the target binding domain that may be used in this embodiment can include GM-CSF.

The method of the present invention requires an endogenous cell population expressing a protein that binds to the CAR protein, aside from the cells to which the polynucleotide encoding the CAR protein is to be introduced. This protein may be the same as or different from the target molecule described above as long as the protein binds to the CAR protein. The target molecule is preferably a target also expressed on cell membrane surface in endogenous cells other than tumor cells.

Furthermore, a ligand that specifically binds to the target, for example, an affibody, a ligand binding domain derived from a natural receptor, a soluble protein/peptide ligand of a receptor (e.g., on tumor cells), or a peptide may be used as the target binding domain.

In the following detailed description, the structure of the CAR protein will be specifically described for the case where human granulocyte-macrophage colony-stimulating factor (GM-CSF) receptor is targeted.

In the case of targeting human GM-CSF receptor, a polypeptide consisting of an amino acid sequence encoded by the open reading frame of human GM-CSF can be used as the target binding domain. Specifically, GM-CSF protein comprising the amino acid sequence of SEQ ID NO: 1 can be used. Alternatively, a polypeptide that consists of an amino acid sequence having 90% or higher sequence identity to the amino acid sequence of SEQ ID NO: 1 and has the ability to bind to the human GM-CSF receptor can be used.

Alternatively, in the case of targeting human GM-CSF receptor, the target binding domain may be a mutant polypeptide (SEQ ID NO: 2) having the substitution of glutamic acid at position 21 in the amino acid sequence represented by SEQ ID NO: 1 with another amino acid, for example, lysine. A mutant also having the substitution of other amino acid residue moieties and a fragment of any of these polypeptides, i.e., a binding fragment may be used as long as the mutant or the fragment retains the ability to bind to the human GM-CSF receptor.

In this context, the phrase "having the ability to bind to the human GM-CSF receptor" means that the association constant to the human GM-CSF receptor is, for example, 1 to 1000 nM or less. The ability to bind can be relatively weak as compared with antigen-antibody binding ability.

Alternatively, in the case of targeting human GM-CSF receptor, the target binding domain can also employ, for example, a single-chain antibody fragment (scFv) derived from a monoclonal antibody having the ability to bind to the human GM-CSF receptor.

The CAR protein can optionally comprise an "extracellular spacer domain" between the target binding domain located extracellularly and the transmembrane domain. The extracellular spacer domain is desirably a sequence that promotes the binding of CAR to the antigen and facilitates signal transduction into a cell. For example, an Fc fragment of an antibody or a fragment or a derivative thereof, a hinge region of an antibody or a fragment or a derivative thereof, a CH2 region of an antibody, a CH3 region of an antibody, an artificial spacer sequence, or a combination thereof can be used.

In one aspect of the present invention, (i) hinge, CH2, and CH3 regions of IgG4, (ii) hinge region of IgG4, (iii) hinge and CH2 of IgG4, (iv) hinge region of CD8a, (v) hinge, CH2 and CH3 regions of IgG1, (vi) hinge region of IgG1, or (vii) hinge and CH2 of IgG1, or a combination thereof can be used as the extracellular spacer domain. For example, a region having the following amino acid sequence (SEQ ID NO: 4) encoded by the nucleotide sequence represented by SEQ ID NO: 3 can be suitably used as the hinge region of IgG1, though the extracellular space domain is not limited thereto.

[Formula 1]

UniProt No.: P01857 (99-110)
<u>EPKSCDKTHTCP</u> PCDPA <u>EPKSPDKTHTCP</u>
Hinge       Spacer       Hinge

A region having an amino acid sequence (SEQ ID NO: 6) encoded by the nucleotide sequence represented by SEQ ID NO: 5 and a region having an amino acid sequence (SEQ ID NO: 8) encoded by the nucleotide sequence represented by SEQ ID NO: 7 can be suitably used as the CH2 region and the CH3 region of IgG1, respectively.

In a preferred aspect, hinge, CH2, and CH3 regions of human IgG1 or a portion thereof can be used as the extracellular spacer domain.

In a preferred aspect, (i) a human IgG1 hinge region (SEQ ID NO: 4) singly, (ii) a combination of a human IgG1 hinge region (SEQ ID NO: 4), CH2 region (SEQ ID NO: 6) and CH3 region (SEQ ID NO: 8), (iii) a combination of a human IgG1 hinge region (SEQ ID NO: 4) and CH3 region (SEQ ID NO: 8), or (iv) a CH3 region (SEQ ID NO: 8) singly can be used as the extracellular spacer domain.

In one aspect of the present invention, a spacer sequence represented by the formula (G4S)n can be used as the artificial spacer sequence for use in the extracellular spacer domain. In the formula, n is 1 to 10, preferably n = 3. For example, the spacer sequence represented by SEQ ID NO: 9 can be suitably used. A spacer having such a spacer sequence is also called peptide linker. Peptide linkers suitably used in the art can be appropriately used in the present invention. In this case, the configuration and chain length of the peptide linker can be properly selected without impairing the function of the resulting CAR protein.

In a further preferred aspect, a combination of a human IgG1 hinge region (SEQ ID NO: 4) and a spacer sequence (SEQ ID NO: 9) of (G4S)3 can be used as the extracellular spacer domain.

The extracellular spacer domain can be appropriately selected from those listed above or further modified on the basis of technical knowledge in the art, and used for the present invention.

Nucleotide sequences encoding the respective amino acid sequences of domains can be linked, inserted into a vector, and expressed in host cells such that the extracellular spacer domain can reside between the target binding domain and the transmembrane domain. Alternatively, the extracellular spacer domain may be modified using a polynucleotide encoding a plasmid CAR protein produced in advance as a template.

The modification of the extracellular spacer domain is useful when, for example, improvement in the CAR gene expression rate of CAR-T cells harboring an introduced polynucleotide encoding CAR in host cells, signal transduction, aging of cells, distribution in tumor, antigen recognition or influence on *in vivo* activity, is taken into consideration.

The CAR protein comprises an extracellular domain comprising a target binding domain and optionally an extracellular spacer domain, a transmembrane domain, and intracellular domain comprising an intracellular signal transduction domain and optionally a costimulatory domain. As well known in the art, the "transmembrane domain" is a domain having affinity to a lipid bilayer constituting a cell membrane, whereas both the extracellular domain and intracellular domain are hydrophilic domains.

The transmembrane domain is not particularly limited as long as the CAR protein can reside on the cell membrane without impairing the functions of the target binding domain and the intracellular signal transduction domain. A polypeptide derived from the same protein as that of a costimulatory domain mentioned later may function as the transmembrane domain. For example, a transmembrane domain such as CD28, CD3ε, CD8α, CD3, CD4 or 4-1BB can be used as the transmembrane domain. For example, human CD28 (Uniprot No.: P10747 (153-179)) can be used as the transmembrane domain. Specifically, a domain having an amino acid sequence (SEQ ID NO: 11) encoded by the nucleotide sequence represented by SEQ ID NO: 10 (NCBI Accession No.: NM_006139.3 (679-759)) can be suitably used as the transmembrane domain.

The CAR protein can optionally comprise a "costimulatory domain". The costimulatory domain specifically binds to a costimulatory ligand, thereby mediating cellular costimulation response such as growth of CAR-T cells, cytokine production, functional differentiation and cell death of target cells, though the cellular costimulation response is not limited thereto. For example, CD27, CD28, 4-1BB (CD137), CD134 (OX40), Dap10, CD27, CD2, CD5, CD30, CD40, PD-1, ICAM-1, LFA-1 (CD11a/CD18), TNFR-1, TNFR-II, Fas, or Lck can be used as the costimulatory domain. For example, human CD28 (Uniprot No.: P10747 (180-220)) or 4-1BB (GenBank: U03397.1) can be used as the costimulatory domain. Specifically, a domain having an amino acid sequence (SEQ ID NO: 13) encoded by the nucleotide sequence represented by SEQ ID NO: 12 (NCBI Accession No.: NM_006139.3 (760-882)) can be suitably used as the costimulatory domain.

The CAR protein comprises an "intracellular signal transduction domain". The intracellular signal transduction domain transduces signals required for exerting the effector function of immunocytes. For example, a human CD3ζ chain, FcγRIII, FcεRI, a cytoplasmic end of Fc receptor, a cytoplasmic receptor having an immunoreceptor tyrosine activation motif (ITAM) or a combination thereof can be used as the intracellular signal transduction domain. For example, a human CD3ζ chain (e.g., nucleotides 299-637 of NCBI Accession No. NM_000734.3) can be used as the intracellular signal transduction domain. Specifically, a domain having an amino acid sequence (SEQ ID NO: 15) encoded by the nucleotide sequence represented by SEQ ID NO: 14 can be suitably used as the intracellular signal transduction domain.

The polynucleotide of interest can be easily produced according to a routine method. Nucleotide sequences encoding the respective amino acid sequences of domains can be obtained from NCBI RefSeq ID or GenBank Accession numbers indicating the amino acid sequences. The polynucleotide of the present invention can be produced using standard molecular biological and/or chemical procedures. For example, nucleic acids can be synthesized on the basis of these nucleotide sequences. Also, DNA fragments obtained using polymerase chain reaction (PCR) from a cDNA library can be combined to produce the polynucleotide of the present invention.

Thus, the polynucleotide encoding the CAR protein can be produced by linking the respective polynucleotides encoding the domains described above. Genetically modified cells can be produced by introducing this polynucleotide to proper cells. Alternatively, the CAR protein may be produced by using a polynucleotide encoding an existing CAR protein having the same constituents except for the target binding domain, as a template, and recombining the target binding domain according to a routine method.

Depending on the purpose, one or more domains, for example, the extracellular spacer domain, can be modified by use of inverse-PCR (iPCR), etc. using a polynucleotide encoding an existing CAR protein as a template. The technique of modifying the extracellular spacer domain is described in, for example, Oncoimmunology, 2016, Vol. 5, No. 12, e1253656.

As used in the present specification, the "polynucleotide" includes, but is not limited to, natural or synthetic DNA and RNA, for example, genomic DNA, cDNA (complementary DNA), mRNA (messenger RNA), rRNA (ribosomal RNA), shRNA (small hairpin RNA), snRNA (small nuclear RNA), snoRNA (small nucleolar RNA), miRNA (microRNA), and/or tRNA.

In the present specification, the term "encoding" means that a predetermined nucleotide sequence has a code for information on the amino acid sequence of a predetermined protein or (poly)peptide, as usually used in the art. In the present specification, both sense and antisense strands are used herein in the context of "encoding".

In the method of the present invention, the introduction of the polynucleotide for preparing genetically modified cells is carried out by a non-viral method based on a transposon method. For the transposon method, a plasmid transposon can be used, and a sleeping beauty transposon system (described in e.g., Huang X, Guo H, et al., Mol Ther. 2008; 16: 580-9; Singh H, Manuri PR, et al., Cancer Res. 2008; 68: 2961-71; Deniger DC, Yu J, et al., PLoS One. 2015; 10: e0128151; Singh H, Moyes JS, et al., Cancer Gene Ther. 2015; 22: 95-100; Hou X, Du Y, et al., Cancer Biol Ther. 2015; 16: 8-16; Singh H, Huls H, et al., Immunol Rev. 2014; 257: 181-90; and Maiti SN, Huls H, et al., J Immunother. 2013; 36: 112-23) or a piggyBac transposon system (described in Nakazawa Y, Huye LE, et al., J Immunother. 2009; 32: 826-36; Galvan DL, Nakazawa Y, et al., J Immunother. 2009; 32: 837-44; Nakazawa Y, Huye LE, et al., Mol Ther. 2011; 19: 2133-43; Huye LE, Nakazawa Y, et al., Mol Ther. 2011; 19: 2239-48; Saha S, Nakazawa Y, et al., J Vis Exp. 2012; (69): e4235; Nakazawa Y, Saha S, et al., J Immunother. 2013; 36: 3-10; Saito S, Nakazawa Y, et al., Cytotherapy. 2014; 16: 1257-69; and Nakazawa et al., Journal of Hematology & Oncology (2016) 9: 27) can be suitably used.

In the case of using a piggyBac transposon system, typically a plasmid carrying a gene encoding piggyBac transposase (in the present specification, referred to as a piggyBac plasmid) and a plasmid having a structure where the polynucleotide encoding the CAR protein is flanked by piggyBac inverted repeat sequences are introduced (transfection). For the transfection, various approaches such as electroporation, nucleofection, lipofection, and calcium phosphate method can be used. Both the plasmids can contain a poly A addition signal sequence, a reporter gene, a selective marker gene, an enhancer sequence, and the like.

The apparatus for use in electroporation is not limited, and, for example, 4D-Nucleofector (Lonza Japan Ltd.) and NEPA21 (Nepa Gene Co., Ltd.) can be used and operated according to their respective instruction manuals.

The method described above enables gene introduction in the range of $1 \times 10^6$ to $2 \times 10^7$ cells.

[Step b)]

The method of the present invention involves providing an endogenous cell population expressing a protein that binds to the CAR expressed by the genetically modified cell population prepared in the step a).

More specifically, the method of the present invention involves providing a cell population derived from PBMCs derived from the mammal expressing a protein that binds to the CAR, wherein said cell population is cultured in the presence of one or more cytokines, wherein said cytokine is GM-CSF and/or IL-4 and wherein said derived PBMCs are PBMCs used without inactivation treatment. Thus, the method of the present invention requires an endogenous cell population to express a protein that binds to the CAR protein. The CAR protein is not particularly limited as long as such an endogenous cell population can be obtained.

The "provision" in this step means that the endogenous cell population to be subjected to coculture in step c) is provided aside from the cell population to which the polynucleotide encoding the CAR is to be introduced in the step a). This step not only comprises subjecting an isolated endogenous cell population directly to coculture in step c) but comprises elevating the proportion of cells expressing the protein of interest and/or culturing the cells for maturation (differentiation). The "isolation" of the endogenous cell population used in the description above does not mean the isolation of only the cells expressing the protein of interest, and rather means that a cell population that comprises the cells expressing the protein of interest and can be used in the step b) is provided,

For example, as described in Examples, a portion of a cell population derived from the same individual can be used as the "cell population comprising T cells" in the step a), and the remaining portion can be used as the "endogenous cell population" for culture in the step b). However, it should be understood that the cell population in the step a) essentially comprises T cells that are activated when the introduced CAR recognizes the target, while for the cell population in the step b), it is important to be a population that enables CAR-specific stimulation using endogenous cells as a feature of the method of the present invention, not exogenous stimulation used in conventional methods.

The expression of the protein that binds to the CAR in the endogenous cell population can be easily confirmed by a routine method. For example, when the CAR protein specifically binds to GM-CSF receptor, the expression of the GM-CSF receptor in the endogenous cell population can be confirmed by flow cytometry analysis using an antibody against the GM-CSF receptor. When the CAR protein specifically binds to CD19, the expression of CD19 in the endogenous cell population can be confirmed by flow cytometry analysis using an anti-CD19 antibody.

In the present specification, the "endogenous cells" refer to cells originally possessed by a mammal of the same species as the origin of the cells to which the polynucleotide encoding the CAR protein is to be introduced. For example, cells collected, isolated, purified or induced from a body fluid such as blood (peripheral blood, umbilical cord blood, etc.) or bone marrow, a tissue or an organ can be used. Peripheral blood mononuclear cells (PBMCs), immunocytes [dendritic cells, B cells, hematopoietic stem cells, macrophages, monocytes, NK cells or hematopoietic cells (neutrophils and basophils)], umbilical blood mononuclear cells, fibroblasts, preadipocytes, stem cells, skin keratinocytes, mesenchymal cells, fatty liver cells, cancer cells or neural stem cells can be used.

In **the present invention PBMCs are** used as the endogenous cells.

Since the "endogenous cells" according to the present invention express a protein that binds to the CAR expressed by the genetically modified cell population prepared in the step a), the endogenous cells can receive CAR-specific stimulation by coculture with the cell population prepared in the step a) and achieve the efficient activation and amplification of T cells. Thus, in the present specification, the CAR stimulation using the endogenous cells is meant to be different from, for example, nonspecific T cell stimulation using an anti-CD3 antibody or an anti-CD28 antibody coating beads or the like, or artificial CAR-specific stimulation with artificial antigen presenting cells (aAPCs) obtained by forcing a tumor cell line such as K562 to express an antigen molecule that binds to CAR or a costimulatory factor. The number of endogenous cells for use in the step b) is appropriately determined from the viewpoint of achieving the activation of T cells by CAR-specific stimulation and suppressing the early exhaustion of T cells by excessive activation. Specific examples are shown in Examples. The endogenous cell population used in the present invention can be used without inactivation treatment based on ultraviolet irradiation treatment or the like.

The endogenous cell population to be provided (isolated and/or cultured) in the step b) is derived from a mammal of the same species as the mammal from which the cell population comprising T cells obtained in the step a) is derived. In a preferred embodiment, the genetically modified cell population and the endogenous cell population are derived from the same individual.

For example, when the CAR protein specifically binds to CD19, a cell population comprising B cells expressing CD19 can be used as the endogenous cell population. For example, when the CAR specifically binds to GM-CSF receptor, a cell population comprising monocytes or cells derived from the monocytes expressing the GM-CSF receptor can be used as the endogenous cell population.

In one embodiment of the present invention, the endogenous cell population comprises cells that are attached to a plate when peripheral blood mononuclear cells (PBMCs) are cultured on the plate. Examples of such cells include monocytes. In another embodiment, the endogenous cell population can be a cell population obtained by culturing PBMCs *in vitro.*

The endogenous cell population is preferably cultured in the presence of one or more cytokines in the step b). The cytokine is GM-CSF and/or IL-4. The GM-CSF and the IL-4 are preferably used at concentrations in the range of, for example, 5 to 20 ng/mL, and, for example, 5 to 20 ng/mL, respectively.

The culture conditions in the step b) are not particularly limited. For example, culture at 37°C in the range of 0 to 10 days is suitable.

[Step c)]

The method of the present invention comprises the step of coculturing the genetically modified cell population obtained in the step a) and the endogenous cell population obtained in the step b).

The step c) can be performed in the presence of one or more cytokines. The cytokine can be, for example, IL-7 and/or IL-15. The IL-7 and the IL-15 are preferably used at concentrations in the range of, for example, 5 to 20 ng/mL, and, for example, 1 to 10 ng/mL, respectively.

The endogenous cell population can be added in one portion or in a plurality of divided portions to the genetically modified cell population.

The culture conditions in the step c) are not particularly limited. For example, culture at 37°C for 1 to 14 days is suitable.

The culture in the step c) may employ a medium supplemented with serum (human serum, fetal bovine serum, artificial serum, etc.). Autologous serum or artificial serum is desirable from the viewpoint of safety for clinical application. In a preferred aspect, artificial serum can be used. The artificial serum can be added to the medium under conditions suitable for culture according to a routine method. The artificial serum is used at a concentration of, for example, 10% or lower. In a preferred aspect, the artificial serum can be used at a concentration in the range of 1 to 5%. The serum can be easily obtained (e.g., provided from Cell Science & Technology Institute, Inc.).

In a preferred embodiment of the present invention, the CAR protein is a protein comprising a binding domain specific for human granulocyte-macrophage colony-stimulating factor (GM-CSF) receptor, and the endogenous cell population is GM-CSF receptor-expressing cells, though the present invention is not limited thereby.

The method of the present invention introduces the polynucleotide by a transposon method without the use of virus vectors and, unlike conventional methods, does not use exogenous stimulation with a viral peptide, a tumor cell line, a monoclonal antibody, beads, or the like. Therefore, the method of the present invention eliminates the need of complicated steps and can also solve safety concerns toward clinical application. Furthermore, the genetically modified cells obtained by the method of the present invention have a high expression rate, also sustain antitumor cell activity for a long period, and have a small proportion of T cells positive to an exhaustion marker PD-1, as compared with conventional methods. The method of the present invention compared with conventional methods has been found to be free from the early exhaustion of T cells associated with cell activation.

Examples

Hereinafter, the present invention will be described further specifically with reference to Examples. However, the present invention is not limited by Examples given below. In Examples given below, an illustrated CAR protein targeting GM-CSF receptor is referred to as "GMR.CAR", and a cell population comprising T cells genetically modified so as to express the CAR protein targeting GM-CSF receptor is referred to as "GMR.CAR-T cells", for the sake of convenience.

[Example 1 Production of GMR.CAR expression plasmid (CAR 001)]

A PCR product of human GM-CSF (coding region of NCBI Accession No.: NM_000758 (SEQ ID NO: 16, nucleotides 33 to 464: prior to stop codon)) linked at its 5' side to an EcoRI site and at its 3' side to an IgG1 hinge-encoding nucleotide sequence and a BclI site was subcloned to a pTA2 cloning vector (Toyobo Co., Ltd.). The pTA2 cloning vector to which the human GM-CSF and IgG1 hinge sequences were subcloned was double-digested with EcoRI and BclI to cleave the human GM-CSF and IgG1 hinge sequences.

Meanwhile, a pSL1190 vector (Amersham plc) was ligated with an anti-CD19 scFv (FMC63; Zola H. et al., Immunol Cell Biol. 1991; 69 (Pt6): 411-412)-CD28-CD3zeta fragment obtained by double-digesting SFG-CD19.CAR (Savoldo et al., J Clin Invest 2011; 121 (5): 1822-1826) with NcoI and SphI, to produce a pSL1190-CD19.CAR vector.

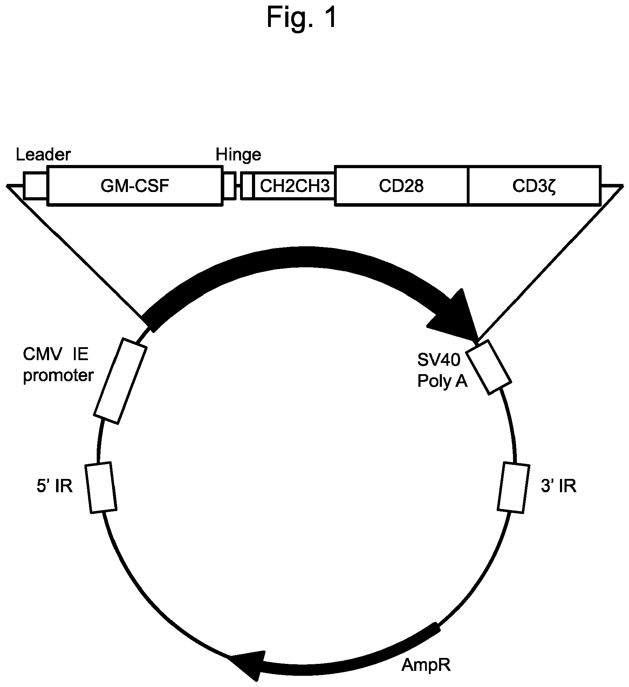

The pSL1190-CD19.CAR vector was double-digested with EcoRI and BamHI and then ligated with the cleaved human GM-CSF and IgG1 hinge sequences mentioned above using compatible sites to produce a pSL1 190-GMR.CAR vector. Further, a pIRII-CD19. CAR vector (Huye et al., Mol Ther 2011; 19 (12): 2239-2248) was double-digested with EcoRI and NotI to remove a CD19.CAR fragment, instead of which a fragment obtained by double-digesting the pSL1 190-GMR.CAR vector with EcoRI and NotI was ligated to obtain a GMR.CAR expression plasmid (CAR 001). Figure 1 shows the vector map of the produced CAR 001.

[Example 2 Production of spacer-modified GMR.CAR expression plasmid (CAR 002)]

A spacer-modified plasmid in which CH2CH3 was partially deleted and (G4S)3 was inserted was produced by inverse-PCR using CAR 001 produced in Example 1 as a template. The PCR primers used in inverse-PCR were designed as given below (SEQ ID NOs: 17 and 18) and synthesized (outsourced to Eurofins Genomics K.K.).

Forward primer:

5'-

GGTGGTGGTGGATCCGGCGGCGGCGGCTCCGGTGGTGGTGGTTCTAAAGATCCCA

AATTTTGGGTGCTGG -3' (SEQ ID NO: 17)

Reverse primer:
5'- TGGGCATGTGTGAGTTTTGTCAGGAGATTTGGGC -3' (SEQ ID NO: 18)

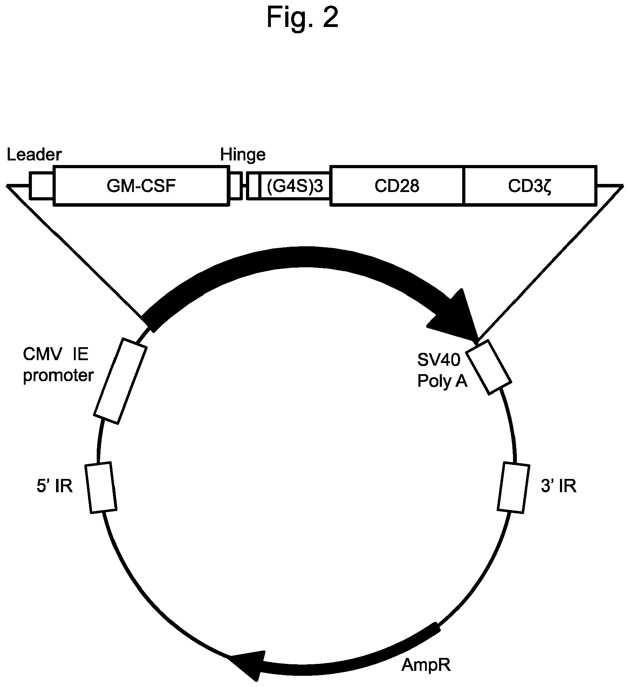

CAR 001 was prepared at 50 ng/$\mu$L and used as a template. Each PCR primer was prepared at a concentration of 0.3 $\mu$M in a reaction solution. Inverse-PCR employed KOD-Plus-Mutagenesis Kit (Toyobo Co., Ltd.), and the reaction composition abided by the protocol attached to the kit. The reaction conditions involved (i) 94°C for 2 minutes, (ii) 98°C for 10 seconds, and (iii) 68°C for 7 minutes with 10 cycles of (ii) and (iii).
After the PCR reaction, an aliquot of the sample was separated by 1 to 1.2% agarose gel electrophoresis to confirm the production of linear DNA having the size of interest. Subsequently, the remaining sample after the PCR reaction was treated with DpnI according to the protocol attached to the kit. Through this reaction, methylated template plasmid CAR 001 was cleaved and eliminated. Then, the linear DNA was self-ligated with Ligation high and T4 Polynucleotide Kinase attached to the kit to form a circular plasmid. Then, *E. coli* DH5$\alpha$ (Toyobo Co., Ltd.) was transformed with the obtained circular plasmid and cultured on an LB agar medium containing 50 $\mu$g/mL ampicillin for approximately 16 hours.
Colonies that appeared were further cultured in an LB liquid medium containing 50 $\mu$g/mL ampicillin for approximately 16 hours. The plasmid was purified from the culture solution of the cultured *E. coli* using QIAprep Spin Miniprep Kit (Qiagen N.V.) and sequenced. A spacer-modified GMR.CAR expression plasmid confirmed to have the modification of the nucleotide sequence of interest was obtained: CAR 002(GMR.CAR dCH2CH3+(G4S)3). The vector map of the produced CAR 002 is shown in Figure 2.

[Example 3 Production of mutant spacer-modified GMR.CAR expression plasmid (CAR 003)]

A mutant spacer-modified plasmid in which E at position 21 of the GM-CSF polypeptide (SEQ ID NO: 1) was substituted with K was produced by inverse-PCR using CAR 002 produced in Example 2 as a template. The PCR primers used in inverse-PCR were designed as given below (SEQ ID NOs: 19 and 20) and synthesized (outsourced to Eurofins Genomics K.K.).

Forward primer:
5'- AAGGCCCGGCGTCTCCTGAACCTG -3' (SEQ ID NO: 19)
Reverse primer:
5'- CTGGATGGCATTCACATGCTCCCAGGG -3' (SEQ ID NO: 20)

CAR 002 was prepared at 50 ng/$\mu$L and used as a template. Each PCR primer was prepared at a concentration of 0.3 $\mu$M in a reaction solution. Inverse-PCR employed KOD-Plus-Mutagenesis Kit (Toyobo Co., Ltd.), and the reaction composition abided by the protocol attached to the kit. The reaction conditions involved (i) 94°C for 2 minutes, (ii) 98°C for 10 seconds, and (iii) 68°C for 7 minutes with 10 cycles of (ii) and (iii).
After the PCR reaction, an aliquot of the sample was separated by 1 to 1.2% agarose gel electrophoresis to confirm the production of linear DNA having the size of interest. Subsequently, the remaining sample after the PCR reaction was treated with DpnI according to the protocol attached to the kit. Through this reaction, methylated template plasmid was cleaved and eliminated. Then, the linear DNA was self-ligated with Ligation high and T4 Polynucleotide Kinase attached to the kit to form a circular plasmid. Then, *E. coli* DH5$\alpha$ (Toyobo Co., Ltd.) was transformed with the obtained circular plasmid and cultured on an LB agar medium containing 50 $\mu$g/mL ampicillin for approximately 16 hours.
Colonies that appeared were further cultured in an LB liquid medium containing 50 $\mu$g/mL ampicillin for ap-

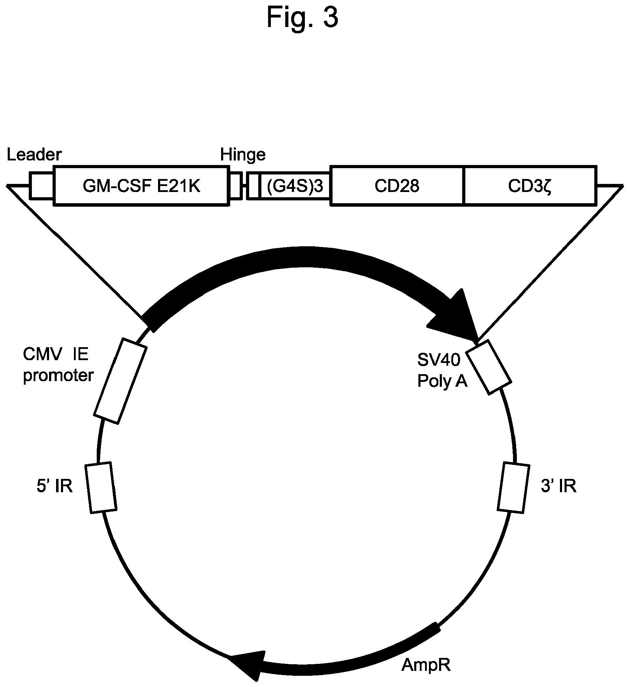

proximately 16 hours. The plasmid was purified from the culture solution of the cultured *E. coli* using QIAprep Spin Miniprep Kit (Qiagen N.V.) and sequenced. A mutant spacer-modified GMR.CAR expression plasmid confirmed to have the modification of the nucleotide sequence of interest was obtained: CAR 003 (E21KGMR.CAR dCH2CH3+(G4S)3). The vector map of the produced CAR 003 is shown in Figure 3.

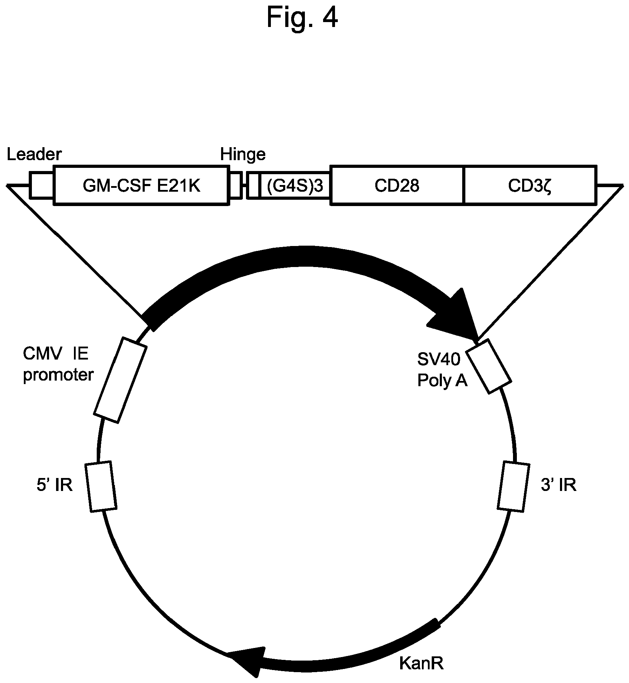

Plasmid modification was carried out by inserting the sequence of kanamycin resistance gene prepared by PCR to CAR 003 and pCMV-piggyBac by seamless cloning so that the resistance gene as a selection marker was changed from ampicillin to kanamycin (outsourced to MediRidge Co., Ltd.). GMR.CAR expression plasmid confirmed by sequencing to have the modification of the nucleotide sequence of interest and pCMV-piggyBac (kan) were obtained: CAR 004 (E21KGMR.CAR dCH2CH3+(G4S)3 kan). The vector map of the produced CAR 004 is shown in Figure 4.

[Example 4 Culture and amplification of GMR.CAR-T cells]

<4-1 Purification of PBMCs>

Peripheral blood was collected from a healthy adult donor and diluted 2-fold with D-PBS (FUJIFILM Wako Pure Chemical Corp.). Then, the diluted peripheral blood was overlayered on Ficoll-Paque PLUS (GE Healthcare Japan Corp.) using SepMate-50 (STEMCELL Technologies Inc.) and centrifuged at $1200 \times g$ for 15 minutes to separate PBMCs. The separated PBMCs were washed twice with D-PB S and purified by centrifugation. The purified PBMCs were used as each of ACE feeder cells (4-2), cells for the electrical introduction of vectors (4-3), and cells for OKT3 blast production (4-4).

<4-2 Production of PBMC ACE feeder cells (Day 0)>

Viral peptide pulse was applied to the PBMCs purified in the preceding section 4-1 using PepTivalor peptide pool(R) (Miltenyi Biotec GmbH). Specifically, the PBMCs were suspended at $12 \times 10^6$ cells in the peptide pool of 100 $\mu$L of D-PBS supplemented with 2 $\mu$L each of 0.05 $\mu$g/$\mu$L AdV5 Hexon, CMV pp65, EBVBZLF1 andEBVEBNA-1 (these four types of peptides are collectively referred to as ACE peptides), and treated with viral peptide pulse at 37°C for 30 minutes in a $CO_2$ incubator. 30 minutes later, 5 mL of D-PBS was added to the PBMCs treated with viral peptide pulse for suspension, followed by UV irradiation for 4 minutes. The UV-irradiated PBMCs (PBMC ACE feeder cells) were collected and centrifuged. The cells thus centrifuged were suspended in ALyS705 (Cell Science & Technology Institute, Inc.) containing 10 ng/mL human IL-7 (Miltenyi Biotec GmbH), 5 ng/mL human IL-15 (Miltenyi Biotec GmbH) and 2% Artificial serum Animal-free (Cell Science & Technology Institute, Inc.), and transferred at $2 \times 10^6$ cells/well/2 mL to a 24-well flat-bottomed multiwell plate for cell culture (FALCON).

<4-3 Electrical introduction of GMR.CAR expression vector and piggyBac vector (Day 0)>

For gene introduction to $15 \times 10^6$ PBMCs purified in the section 4-1, 7.5 $\mu$g each of the CAR 004 plasmid and the pCMV-piggyBac (kan) plasmid produced in Example 3 was added to the cells, which were then suspended in ALyS705 containing 2% Artificial serum Animal-free. The suspended cells were loaded in NEPA Electroporation Cuvettes 2 mm Gap (Nepa Gene Co., Ltd.), and the resistance value of the suspension was measured using a gene introduction apparatus NEPA21 (Nepa Gene Co., Ltd.). In this operation, the amount of the liquid was adjusted using ALyS705 containing 2% Artificial serum Animal-free such that the resistance value fell within a proper range (0.038 to 0.046 k$\Omega$) recommended by the manufacturer. After the adjustment, electrical introduction was carried out within a range settable by NEPA21.

The total amount of the cells after the electrical introduction was added to the 24-well flat-bottomed multiwell plate for cell culture containing the PBMC ACE feeder cells produced in the section 4-2 in advance to start culture in a $CO_2$ incubator at 37°C. Mock-T cells electrically stimulated without plasmid addition were similarly cultured. The cells were cultured for 7 days in the 24-well flat-bottomed multiwell plate for cell culture, and during this period, medium replacement and the addition of IL-7 and IL-15 were appropriately performed.

<4-4 Production of OKT3 blast (Days 0 to 7)>

On Day 0, an anti-human CD3 antibody (Miltenyi Biotec GmbH) and an anti-human CD28 antibody (Miltenyi Biotec GmbH) each adjusted at 1 $\mu$g/mL with D-PBS were added at 500 $\mu$L/well to a 24-well non-treatment flat-bottomed plate (FALCON). The plate was left at 37°C for 2 hours in a $CO_2$ incubator to immobilize the antibodies onto the plate. 2 hours later, the supernatant was discarded to produce an antibody-immobilized plate.

The PBMCs purified in the section 4-1 were suspended in ALyS705 containing 5 ng/mL human IL-15 and 2% Artificial serum Animal-free. The suspended PBMCs were inoculated at $1.5 \times 10^6$ cells/well/2 mL to the plate on which the antibodies were immobilized in advance, to start culture while stimulating T cells.

On Day 3, an antibody-immobilized plate for re-stimulation of OKT3 blast was produced. Specifically, an anti-human CD3 antibody and an anti-human CD28 antibody each adjusted to 1 μg/mL with D-PBS were added at 500 μL/well to a 24-well non-treatment flat-bottomed plate and incubated overnight at 4°C to immobilize the antibodies onto the plate.

Meanwhile, OKT3 blast stimulated from Day 0 was transferred to an antibody-unimmobilized 24-well flat-bottomed multiwell plate for cell culture and thereby was not stimulated temporarily.

On Day 4, a supernatant was removed from the plate subjected to the antibody immobilization on the previous day. OKT3 blast was added thereto to start the re-stimulation of the OKT3 blast with the anti-human CD3 antibody and the anti-human CD28 antibody. The re-stimulation was carried out until Day 7. During this period, medium replacement and the addition of IL-15 were appropriately performed.

<4-5 Production of OKT3 ACE feeder cells (Day 7)>

OKT3 blast produced in the section 4-4 was subjected to viral peptide pulse with the PepTivator peptide pool and UV irradiation similarly as in the section 4-2 to prepare OKT3 ACE feeder cells. These OKT3 ACE feeder cells were collected, suspended at $5 \times 10^6$ cells in 30 mL of ALyS705 containing 10 ng/mL human IL-7, 5 ng/mL human IL-15 and 2% Artificial serum Animal-free, and transferred to G-Rex 6-well cell culture plate (Wilson Wolf Manufacturing Corporation).

<4-6 Culture and amplification of CAR-T cells (Days 7 to 14)>

The total amount of the cells after the electrical introduction in the section 4-3 was transferred to the G-Rex 6-well cell culture plate to which the OKT3 ACE feeder cells were inoculated in the section 4-5. The GMR.CAR-T cells were cultured and amplified until Day 14, and during this period, medium replacement and the addition of IL-7 and IL-15 were appropriately performed. GMR.CAR-T cells obtained by electrical introduction operation to PBMCs using the expression plasmid CAR 004 and the culture and amplification of T cells were designated as CAR-T 004.

<4-7 Evaluation of GMR.CAR expression rate (Day 14)>

The GMR.CAR-T cells cultured and amplified until Day 14 were evaluated for the expression rate of GMR.CAR by flow cytometry analysis. 100 μL of the cell culture suspension in the preceding section 4-6 was collected and centrifuged, and the resulting cells were suspended by the addition of 5 μL of PE Anti-human GM-CSF antibody (Miltenyi Biotec GmbH) and 5 μL of APC Anti-human CD3 antibody (Miltenyi Biotech GmbH), and subjected to antibody labeling reaction at 4°C for 20 minutes in the dark. 20 minutes later, the cells were washed with D-PBS and precipitated by centrifugation. After removal of the supernatant, the cells were resuspended in an appropriate amount of D-PBS. This sample was analyzed using FACSCanto II (Becton, Dickinson and Company) and FLOWJO (Becton, Dickinson and Company) to measure the expression rate of GMR.CAR with GM-CSF/CD3 positivity as an index.

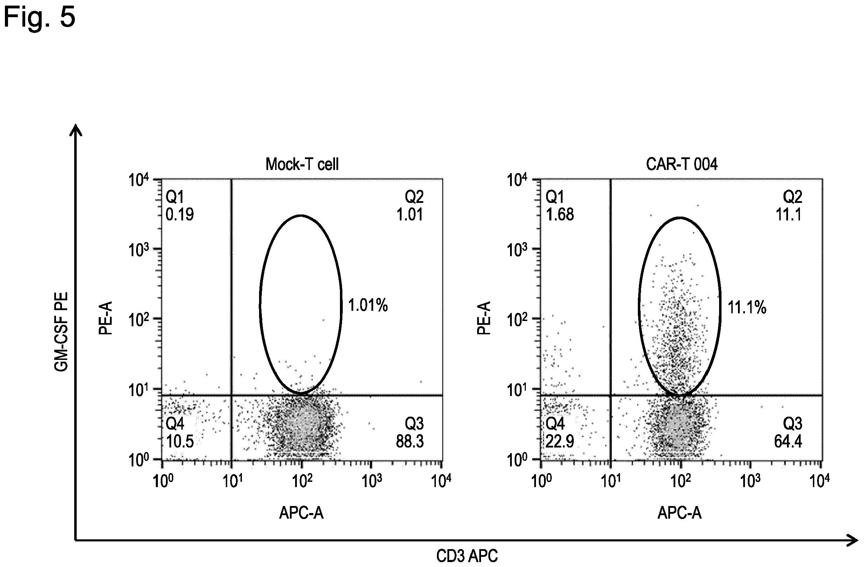

The expression analysis results of CAR-T 004 on Day 14 are shown in Figure 5. The GMR.CAR expression rate was 11.1%.

[Example 5 Culture and amplification of GMR.CAR-T cells using endogenous cells (method A)]

<5-1 Purification of PBMCs (Day 0)>

Peripheral blood was collected from the same healthy adult donor as in Example 4 and diluted 2-fold with D-PBS (FUJIFILM Wako Pure Chemical Corp.). Then, the diluted peripheral blood was overlayered on Ficoll-Paque PLUS (GE Healthcare Japan Corp.) using SepMate-50 (STEMCELL Technologies Inc.) and centrifuged at $1200 \times g$ for 15 minutes to separate PBMCs. The separated PBMCs were washed twice with D-PBS and purified by centrifugation. The purified PBMCs were used as each of cells for the electrical introduction of vectors (5-3), and endogenous cells for CAR stimulation (5-4 and 5-5).

<5-2 Separation of purified PBMCs (Day 0)>

The PBMCs purified in the section 5-1 were suspended in D-PBS and inoculated at $20 \times 10^6$ cells/well/2 mL to a 24-well flat-bottomed multiwell plate for cell culture (FALCON). The inoculated cells were left standing at 37°C for 2 hours in a $CO_2$ incubator. Then, floating cells were recovered together with the culture supernatant, and electrical introduction was carried out as described below in the section 5-3.

Meanwhile, cells attached to the plate were cultured in 2 mL of ALyS705 (Cell Science & Technology Institute, Inc.) containing 10 ng/mL human IL-7 (Miltenyi Biotec GmbH), 5 ng/mL human IL-15 (Miltenyi Biotec GmbH) and 2% Artificial serum Animal-free (Cell Science & Technology Institute, Inc.), and used as endogenous cells for CAR stimulation in operation in the section 5-4 described below.

<5-3 Electrical introduction of GMR.CAR expression vector and piggyBac vector (Day 0)>

For gene introduction to the floating cells recovered in the section 5-2, 7.5 μg each of the CAR 004 plasmid and the pCMV-piggyBac (kan) plasmid was added to the cells, which were then suspended in ALyS705 containing 2% Artificial serum Animal-free. The suspended cells were loaded in NEPA Electroporation Cuvettes 2 mm Gap (Nepa Gene Co., Ltd.), and the resistance value of the suspension was measured using a gene introduction apparatus NEPA21 (Nepa Gene Co., Ltd.). In this operation, the amount of the liquid was adjusted using ALyS705 containing 2% Artificial serum Animal-free such that the resistance value fell within a proper range (0.038 to 0.046 kΩ) recommended by the manufacturer. After the adjustment, electrical introduction was carried out using the same set value as in Example 4.

<5-4 Initial CAR stimulation and culture of cells after gene introduction (Days 0 to 7)>

The total amount of the cells after the electrical introduction in the section 5-3 was transferred to the 24-well flat-bottomed multiwell plate for cell culture attached with the endogenous cells for CAR stimulations provided in the section 5-2 in advance to start culture in a $CO_2$ incubator at 37°C. Mock-T cells electrically stimulated without plasmid addition were similarly cultured. During a culture period of 7 days, medium replacement and the addition of IL-7 and IL-15 were appropriately performed.

<5-5 Culture of cells for CAR stimulations using PBMCs (Days 0 to 7)>

The PBMCs purified in the section 5-1 were suspended in ALyS705 containing 10 ng/mL human GM-CSF (Miltenyi Biotec GmbH) and 10 ng/mL human IL-4 (Miltenyi Biotec GmbH) and inoculated at $5 \times 10^6$ cells/well/15 mL to G-Rex 6-well cell culture plate (Wilson Wolf Manufacturing Corporation) to start culture in a $CO_2$ incubator at 37°C. During a culture period of 7 days, the addition of GM-CSF and IL-4 were appropriately performed.

<5-6 Second CAR stimulation and culture and amplification of cells after gene introduction (Days 7 to 13)>

A supernatant was removed from the G-Rex 6-well cell culture plate incubated for 7 days in the section 5-5, and 30 mL of ALyS705 containing 10 ng/mL human IL-7, 5 ng/mL human IL-15 and 2% Artificial serum Animal-free was added to each well. The total amount of the cells cultured for 7 days in the 24-well flat-bottomed multiwell plate for cell culture was further transferred to the G-Rex 6-well cell culture plate to carry out the second CAR stimulation of the cells after the gene introduction. Then, the cells were further cultured and amplified for 6 days and during this period, medium replacement and the addition of IL-7 and IL-15 were appropriately performed. The expression rate of GMR.CAR in the GMR.CAR-T cells cultured and amplified until Day 13 was measured by the method given below. In this Example, GMR.CAR-T cells obtained by the culture and amplification of the GMR.CAR-T cells stimulated with CAR using the endogenous cells and the expression plasmid CAR 004 were designated as CAR-T 004-A.

<5-7 Evaluation of GMR.CAR expression rate (Day 13)>

The GMR.CAR-T cells cultured and amplified until Day 13 were evaluated for the expression rate of GMR.CAR by flow cytometry analysis. 100 μL of the cell culture suspension in the section 5-6 was collected and centrifuged, and the resulting cells were suspended by the addition of 5 μL of PE Anti-human GM-CSF antibody (Miltenyi Biotec GmbH) and 5 μL of APC Anti-human CD3 antibody (Miltenyi Biotech GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCanto II (Becton, Dickinson and Company) and FLOWJO (Becton, Dickinson and Company) to measure the expression rate of GMR.CAR with GM-CSF/CD3 positivity as an index.

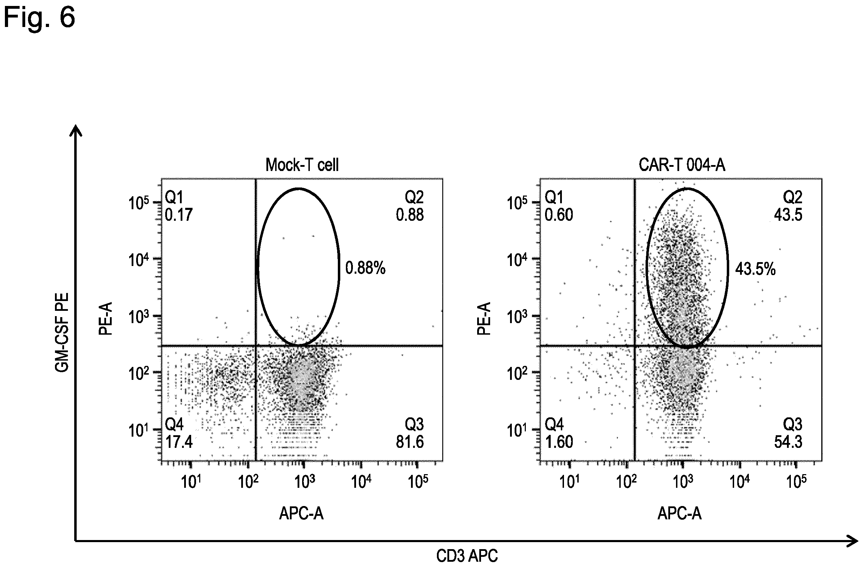

The expression analysis results of CAR-T 004-A on Day 13 are shown in Figure 6. The GMR.CAR expression rate was 43.5%, which was higher than the expression rate of 0.88% in the Mock-T cells. The expression rate was also drastically improved as compared with the results of Example 4.

[Example 6 Culture and amplification of GMR.CAR-T cells using endogenous cells (method B)]

<6-1 Purification of PBMCs (Day 0)>

Peripheral blood was collected from the same healthy adult donor as in Examples 4 and 5 and diluted 2-fold with D-PBS (FUJIFILM Wako Pure Chemical Corp.). Then, the diluted peripheral blood was overlayered on Ficoll-Paque PLUS (GE Healthcare Japan Corp.) using SepMate-50 (STEMCELL Technologies Inc.) and centrifuged at $1200 \times$ g for 15 minutes to separate PBMCs. The separated PBMCs were washed twice with D-PBS and purified by centrifugation. The purified PBMCs were used as each of cells for the electrical introduction of vectors (6-2), and endogenous cells for CAR stimulations (6-3).

<6-2 Electrical introduction and culture of GMR.CAR expression vector and piggyBac vector (Days 0 to 3)>

For gene introduction to $15 \times 10^6$ PBMCs purified in the section 6-1, 7.5 $\mu$g each of the CAR 004 plasmid and the pCMV-piggyBac (kan) plasmid was added to the cells, which were then suspended in ALyS705 (Cell Science & Technology Institute, Inc.) containing 2% Artificial serum Animal-free (Cell Science & Technology Institute, Inc.). The suspended cells were loaded in NEPA Electroporation Cuvettes 2 mm Gap (Nepa Gene Co., Ltd.), and the resistance value of the suspension was measured using a gene introduction apparatus NEPA21 (Nepa Gene Co., Ltd.). In this operation, the amount of the liquid was adjusted using ALyS705 containing 2% Artificial serum Animal-free such that the resistance value fell within a proper range (0.038 to 0.046 k$\Omega$) recommended by the manufacturer. After the adjustment, electrical introduction was carried out using the same set value as in Examples 4 and 5.
The total amount of the cells after the electrical introduction was suspended in 2 mL of ALyS705 containing 10 ng/mL human IL-7 (Miltenyi Biotec GmbH), 5 ng/mL human IL-15 (Miltenyi Biotec GmbH) and 2% Artificial serum Animal-free, inoculated to a 24-well flat-bottomed multiwell plate for cell culture (FALCON), and cultured for 3 days in a $CO_2$ incubator at 37°C. Mock-T cells electrically stimulated without plasmid addition were similarly cultured.

<6-3 Culture of cells for CAR stimulations using PBMCs (Days 0 to 3)>

The PBMCs purified in the section 6-1 were suspended in ALyS705 containing 10 ng/mL human GM-CSF (Miltenyi Biotec GmbH) and 10 ng/mL human IL-4 (Miltenyi Biotec GmbH), inoculated at $5 \times 10^6$ cells/well/15 mL to G-Rex 6-well cell culture plate (Wilson Wolf Manufacturing Corporation), and cultured for 3 days in a $CO_2$ incubator at 37°C.

<6-4 Initial CAR stimulation and culture and amplification of cells after gene introduction (Days 3 to 10)>

A supernatant was removed from the G-Rex 6-well cell culture plate incubated for 3 days in the section 6-3, and 30 mL of ALyS705 containing 10 ng/mL human IL-7, 5 ng/mL human IL-15 and 2% Artificial serum Animal-free was added to each well. The total amount of the cells after the gene introduction of the section 6-2 cultured for 3 days in the 24-well flat-bottomed multiwell plate for cell culture was further transferred to the G-Rex 6-well cell culture plate to carry out the CAR stimulation of the cells after the gene introduction. Then, the cells were further cultured for 7 days and during this period, medium replacement and the addition of IL-7 and IL-15 were appropriately performed. The expression rate of GMR.CAR in the GMR.CAR-T cells cultured and amplified until Day 10 was measured by the method given below. GMR.CAR-T cells obtained by the culture and amplification of the GMR.CAR-T cells stimulated with CAR by a more convenient method than that of Example 5 using the endogenous cells and the expression plasmid CAR 004 were designated as CAR-T 004-B.

<6-5 Evaluation of GMR.CAR expression rate (Day 10)>

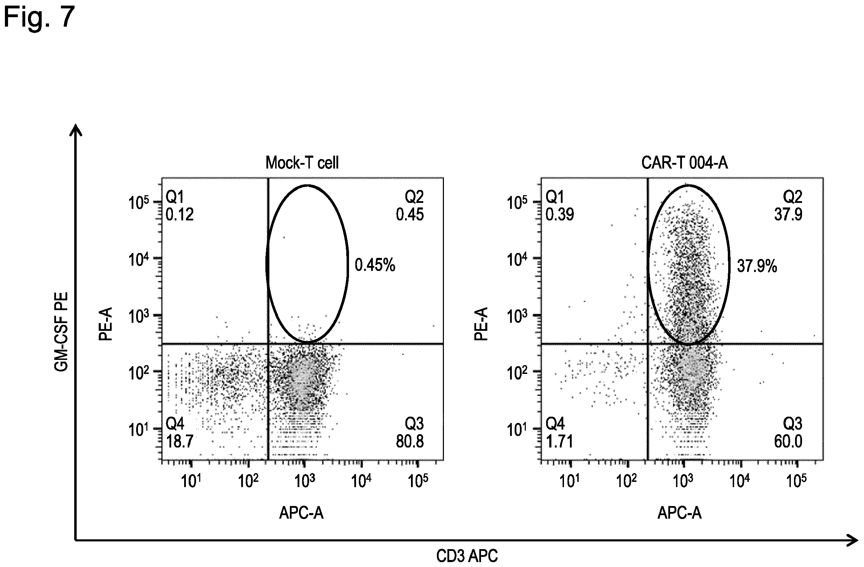

The GMR.CAR-T cells cultured and amplified until Day 10 were evaluated for the expression rate of GMR.CAR by flow cytometry analysis. 100 $\mu$L of the cell culture suspension in the section 6-4 was collected and centrifuged, and the resulting cells were suspended by the addition of 5 $\mu$L of PE Anti-human GM-CSF antibody (Miltenyi Biotec GmbH) and 5 $\mu$L of APC Anti-human CD3 antibody (Miltenyi Biotech GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCanto II (Becton, Dickinson and Company) and FLOWJO (Becton, Dickinson and Company) to measure the expression rate of GMR.CAR with GM-CSF/CD3 positivity as an index.
The expression analysis results of CAR-T 004-B on Day 10 are shown in Figure 7. The GMR.CAR expression rate was 37.9%, which was higher than the expression rate of 0.45% in the Mock-T cells. The expression rate was also drastically improved as compared with the results of Example 4. This method simplifies steps and reduces the number of culture days as compared with the method A and however, was able to provide an expression rate equivalent thereto.

[Example 7 Evaluation of sustained antitumor cell activity of GMR.CAR-T]

CAR-T cells expected to be effective in adoptive immunotherapy are required to have sustained cytotoxic activity. Thus, sustained antitumor cell activity was evaluated using CAR-T 004-B produced in Example 6.

In order to evaluate CAR-T 004-B cultured and amplified until Day 10 for its sustained cytotoxic activity against tumor cells, a test was conducted by coculture with tumor cells for a long period. Specifically, AML tumor cell line THP-1 cells (American Type Culture Collection) were suspended at $2.5 \times 10^5$ cells/mL in RPMI 1640 Medium, GlutaMAX Supplement (Thermo Fisher Scientific Inc.) containing 10% FBS (Corning Inc.) and inoculated at 1 mL/well to a 24-well treatment culture plate. Likewise, CAR-T 004-B was suspended at $2.5 \times 10^5$ cells/mL in RPMI 1640 Medium, GlutaMAX Supplement containing 10% FBS and inoculated at 1 mL/well, from above, to the 24-well treatment culture plate inoculated with the THP-1 cells in advance. A plurality of such wells were provided, and coculture with the tumor cells was started.

4 days after the start of coculture, all the intrawell cells were recovered from only one well among the plurality of wells and centrifuged. The centrifuged cells were suspended by the addition of 5 $\mu$L of APC Anti-Human CD3 antibody (Miltenyi Biotec GmbH) and 5 $\mu$L of PE Anti-Human CD33 antibody (Miltenyi Biotec GmbH), and subjected to antibody labeling reaction at 4°C for 20 minutes in the dark. 20 minutes later, the cells were washed with an appropriate amount of D-PBS and precipitated by centrifugation. After complete removal of the supernatant, the cells were resuspended in 450 $\mu$L of D-PBS, and 50 $\mu$L of CountBright absolute counting beads (Invitrogen Corp.) was further added thereto. This sample was analyzed using FACS Canto II (Becton, Dickinson and Company) and FLOWJO (Becton, Dickinson and Company). The number of CD33-positive (CAR-T 004-B) cells and the number of CD33-positive (tumor) cells were evaluated on the basis of the counting beads.

When the tumor cells were killed (the number of tumor cells was decreased) as compared with those at the start of coculture, 1 mL of a supernatant was removed from each of the remaining wells, and THP-1 cells prepared at $2.5 \times 10^5$ cells/mL with RPMI 1640 Medium, GlutaMAX Supplement containing 10% FBS were added 1 mL/well to start coculture with fresh tumor cells. This series of operations was repeated for a culture period of 4 days or 3 days, and the coculture was terminated at the time when the number of tumor cells was increased as compared with that at the start of coculture.

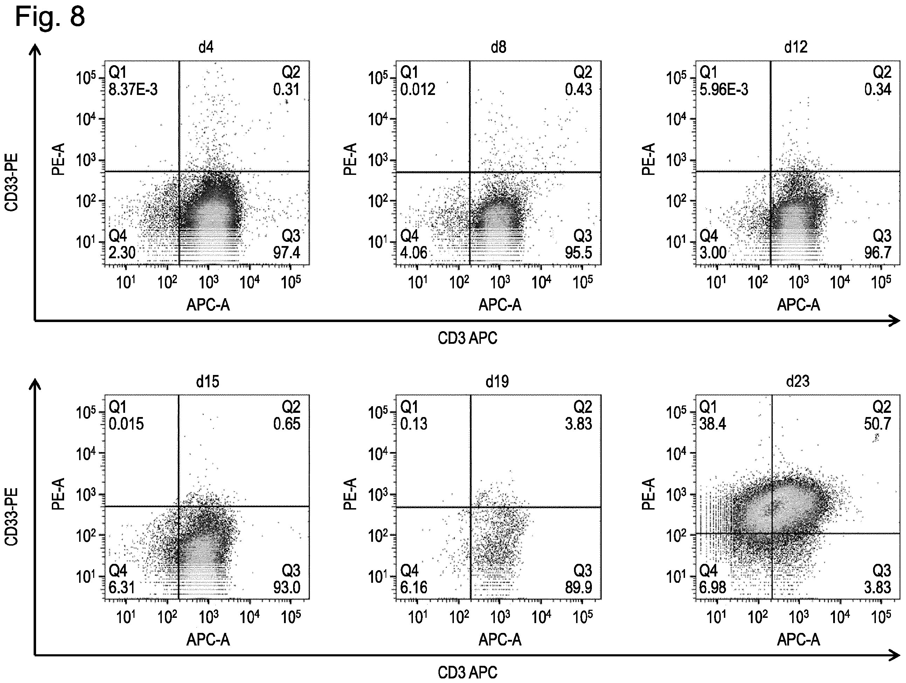

Figure 8 shows the results of evaluating antitumor cell activity on days 4, 8, 12, 15, 19, and 23 after the start of coculture. These results indicate that CAR-T 004-B kills THP-1 cells a plurality of times. Thus, CAR-T 004-B was confirmed to have a sustained killing effect.

[Example 8 Culture and amplification of GMR.CAR-T cells using endogenous cells (method C)]

<8-1 Purification of PBMCs (Day 0)>

Peripheral blood was collected from the same healthy adult donor as in Examples 4, 5 and 6 and diluted 2-fold with D-PBS (FUJIFILM Wako Pure Chemical Corp.). Then, the diluted peripheral blood was overlayered on Ficoll-Paque PLUS (GE Healthcare Japan Corp.) using SepMate-50 (STEMCELL Technologies Inc.) and centrifuged at $1200 \times$ g for 15 minutes to separate PBMCs. The separated PBMCs were washed twice with D-PBS and purified by centrifugation. The purified PBMCs were used as each of cells for the electrical introduction of vectors (8-2), and endogenous cells for CAR stimulations (8-3).

<8-2 Electrical introduction and culture of GMR.CAR expression vector and piggyBac vector (Days 0 to 3)>

For gene introduction to $20 \times 10^6$ PBMCs purified in the section 8-1, 7.5 $\mu$g each of the CAR 004 plasmid and the pCMV-piggyBac (kan) plasmid was added to the cells, which were then suspended in ALyS705 (Cell Science & Technology Institute, Inc.) containing 5% Artificial serum Animal-free (Cell Science & Technology Institute, Inc.). The suspended cells were loaded in NEPA Electroporation Cuvettes 2 mm Gap (Nepa Gene Co., Ltd.), and the resistance value of the suspension was measured using a gene introduction apparatus NEPA21 (Nepa Gene Co., Ltd.). In this operation, the amount of the liquid was adjusted using ALyS705 containing 5% Artificial serum Animal-free such that the resistance value fell within a proper range (0.038 to 0.046 k$\Omega$) recommended by the manufacturer. After the adjustment, electrical introduction was carried out using the same set value as in Examples 4, 5 and 6.

The total amount of the cells after the electrical introduction was suspended in 2 mL of ALyS705 containing 10 ng/mL human IL-7 (Miltenyi Biotec GmbH), 5 ng/mL human IL-15 (Miltenyi Biotec GmbH) and 5% Artificial serum Animal-free, inoculated to a 24-well flat-bottomed multiwell plate for cell culture (FALCON), and cultured for 3 days in a $CO_2$ incubator at 37°C. Mock-T cells electrically stimulated without plasmid addition were similarly cultured.

<8-3 Culture of cells for CAR stimulations using PBMCs (Days 0 to 3)>

The PBMCs purified in the section 8-1 were suspended in ALyS705, inoculated at $5 \times 10^6$ cells/well/15 mL to G-Rex 6-well cell culture plate (Wilson Wolf Manufacturing Corporation), and cultured for 3 days in a $CO_2$ incubator at 37°C.

<8-4 Initial CAR stimulation and culture and amplification of cells after gene introduction (Days 3 to 11)>

A supernatant was removed from the G-Rex 6-well cell culture plate incubated for 3 days in the section 8-3, and 30 mL of ALyS705 containing 10 ng/mL human IL-7, 5 ng/mL human IL-15 and 5% Artificial serum Animal-free was added to each well. The total amount of the cells after the gene introduction of the section 8-2 cultured for 3 days in the 24-well flat-bottomed multiwell plate for cell culture was further transferred to the G-Rex 6-well cell culture plate to carry out the CAR stimulation of the cells after the gene introduction. Then, the cells were further cultured for 8 days and during this period, medium replacement and the addition of IL-7 and IL-15 were appropriately performed. The expression rate of GMR.CAR in the GMR.CAR-T cells cultured and amplified until Day 11 was measured by the method given below. Unlike Example 6, the endogenous cells were cultured without the addition of cytokines such as IL-4 or GM-CSF. GMR.CAR-T cells obtained by the culture and amplification of the GMR.CAR-T cells stimulated with CAR by a more convenient method than that of Example 5 and the expression plasmid CAR 004 were designated as CAR-T 004-C.

<8-5 Evaluation of GMR.CAR expression rate (Day 11)>

The GMR.CAR-T cells cultured and amplified until Day 11 were evaluated for the expression rate of GMR.CAR by flow cytometry analysis. 100 μL of the cell culture suspension in the section 8-4 was collected and centrifuged, and the resulting cells were suspended by the addition of 5 μL of PE Anti-human GM-CSF antibody (Miltenyi Biotec GmbH) and 5 μL of APC Anti-human CD3 antibody (Miltenyi Biotech GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCanto II (Becton, Dickinson and Company) and FLOWJO (Becton, Dickinson and Company) to measure the expression rate of GMR.CAR with GM-CSF/CD3 positivity as an index.

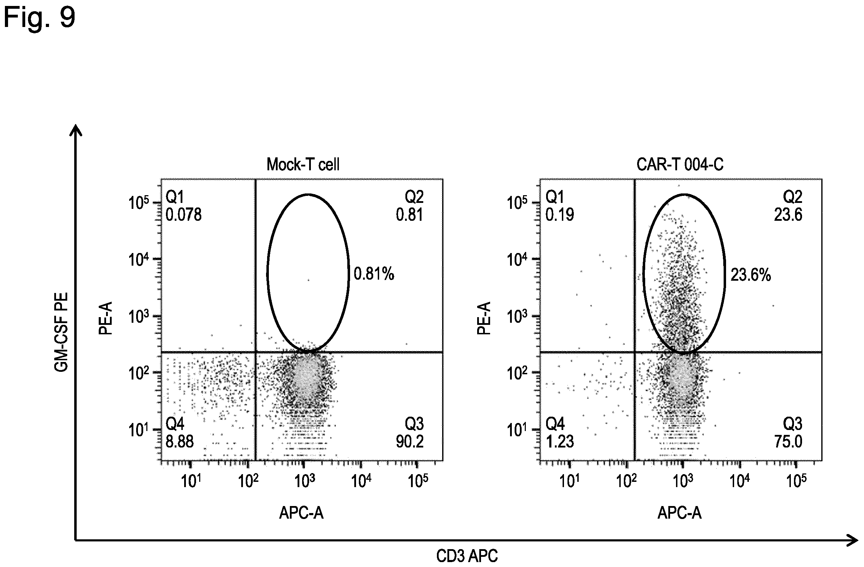

The expression analysis results of CAR-T 004-C on Day 11 are shown in Figure 9. The GMR.CAR expression rate was 23.6%, which was higher than the expression rate of 0.81% in the Mock-T cells. The expression rate was also drastically improved as compared with the results of Example 4.

[Example 9 Phenotype analysis of GMR.CAR-expressing T cells]

CAR-T 004-A produced in Example 5 and CAR-T 004-B produced in Example 6 were cultured until Day 14, and these GMR.CAR-T cells were analyzed for their phenotype. An exhaustion rate with PD-1-positive rate as an index and the proportion of a stem cell memory fraction with CD45RA/CCR7-positive rate as an index were calculated by the phenotype analysis.

$1 \times 10^6$ cells were collected and centrifuged, and the resulting cells were suspended in 100 μL of D-PBS (FUJIFILM Wako Pure Chemical Corp.) containing 3% FBS (Corning Inc.). Then, 5 μL of human BD Fc Block (Becton, Dickinson and Company) was added thereto, and the cells were incubated on ice for 10 minutes to carry out Fc receptor blocking treatment. Further, 50 μL of Brilliant Stain Buffer (Becton, Dickinson and Company) supplemented with 5 μL each of PE-Cy7 Anti-human CD45RA antibody (Becton, Dickinson and Company), BV421 Anti-human CCR7 antibody (BioLegend, Inc.), AF647 Anti-human PD-1 antibody (Becton, Dickinson and Company), and PE Anti-human GM-CSF antibody (Miltenyi Biotec GmbH) was added to the cell suspension after the blocking treatment and subjected to antibody labeling reaction at 4°C for 20 minutes in the dark.

$1 \times 10^6$ cells were reacted with PE-Cy7 Isotype control antibody (Becton, Dickinson and Company), AF647 Isotype control antibody (Becton, Dickinson and Company), and BV421 Isotype control antibody (Becton, Dickinson and Company) by the same procedures as above, and used as an isotype control.

20 minutes later, the cells were washed with an appropriate amount of D-PBS containing 3% FBS and precipitated by centrifugation. After removal of the supernatant, the cells were resuspended in an appropriate amount of D-PBS containing 3% FBS. This sample was measured and analyzed using FACSCelesta (Becton, Dickinson and Company) and FLOWJO (Becton, Dickinson and Company) to evaluate PD-1-positive rate (exhaustion rate) and CD45RA/CCR7-positive rate (proportion of a stem cell memory fraction).

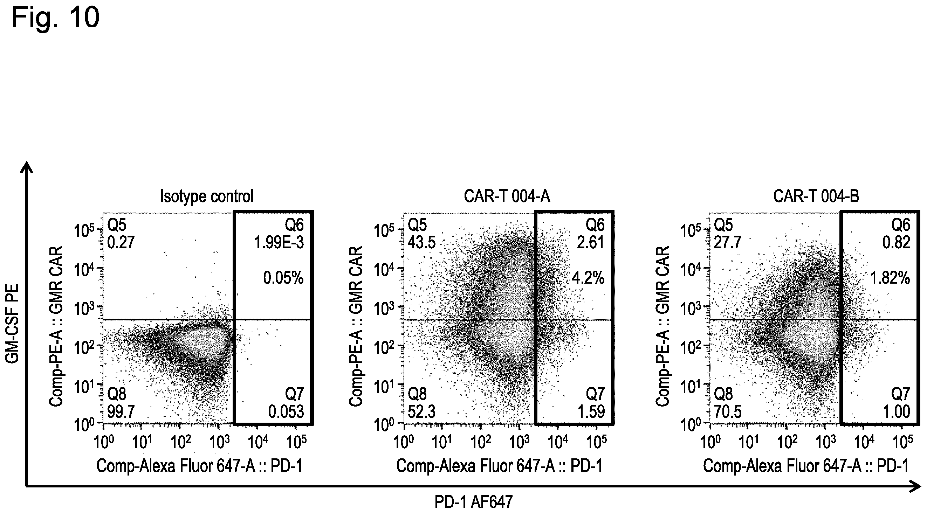

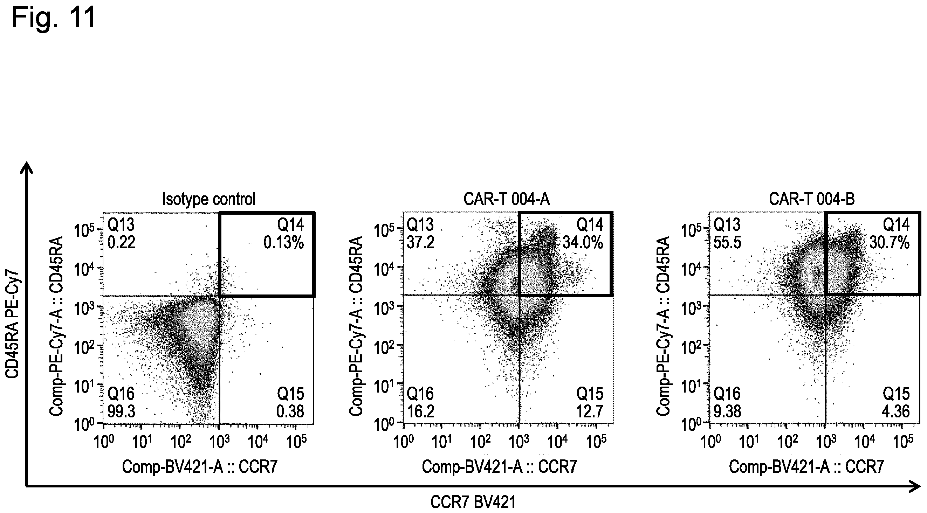

As a result, as shown in Figure 10, the PD-1-positive rates of CAR-T 004-A and CAR-T 004-B were 4.2% and 1.82%, respectively. Thus, the proportion of exhausted cells with CAR stimulation was small. As shown in Figure 11, the CD45RA/CCR7-positive rates of CAR-T 004-A and CAR-T 004-B were 34.0% and 30.7%, respectively.

Industrial Applicability

According to the present invention, genetically modified cells expressing a CAR protein that can be used in adoptive immunotherapy can be produced by a convenient method without the use of viruses. Thus, the present invention provides a safer method for producing genetically modified cells at reduced cost as compared with conventional methods.

**Claims**

1. A method for producing genetically modified mammalian cells, comprising the steps of:

a) introducing a polynucleotide encoding a chimeric antigen receptor (CAR) protein to a cell population comprising T cells derived from a mammal by a transposon method to obtain a genetically modified cell population;
b) providing a cell population derived from PBMCs derived from the mammal expressing a protein that binds to the CAR, wherein said cell population is cultured in the presence of one or more cytokines, wherein said cytokine is GM-CSF and/or IL-4 and wherein said derived PBMCs are PBMCs used without inactivation treatment; and
c) coculturing the genetically modified cell population of the step a) and the cell population of the step b).

2. The method according to claim 1, wherein the step c) is performed in the presence of one or more cytokines.

3. The method according to claim 2, wherein the cytokine in the step c) is IL-7 and/or IL-15.

4. The method according to any one of claims 1 to 3 wherein the transposon method is a piggyBac method.

5. The method according to any one of claims 1 to 4, wherein the genetically modified cell population and the cell population are derived from the same individual.

6. The method according to any one of claims 1 to 5, wherein the CAR protein is a protein comprising a binding domain specific for human granulocyte-macrophage colony-stimulating factor (GM-CSF) receptor, and the endogenous cell population is GM-CSF receptor-expressing cells.

**Patentansprüche**

1. Verfahren zur Herstellung von genetisch veränderten Säugerzellen, umfassend die Schritte:

a) Einführen eines Polynucleotids, das ein Chimärer-Antigenrezeptor (chimeric antigen receptor, CAR)-Protein codiert, in eine Zellpopulation, die T-Zellen umfasst, die von einem Säuger stammen, durch ein Transposonverfahren, um eine genetisch veränderte Zellpopulation zu erhalten;
b) Bereitstellen einer Zellpopulation, die von PBMCs stammt, die von einem Säuger stammen, und ein Protein exprimieren, das an den CAR bindet, wobei die Zellpopulation in der Anwesenheit eines Cytokins oder mehrerer Cytokine gezüchtet wird, wobei das Cytokin GM-CSF und/oder IL-4 ist und wobei die abgeleiteten PBMCs PBMCs sind, die ohne Inaktivierungsbehandlung verwendet werden; und
c) Co-Züchten der genetisch veränderten Zellpopulation aus Schritt a) und der Zellpopulation aus Schritt b).

2. Verfahren nach Anspruch 1, wobei der Schritt c) in der Anwesenheit eines Cytokins oder mehrerer Cytokine durchgeführt wird.

3. Verfahren nach Anspruch 2, wobei das Cytokin im Schritt c) IL-7 und/oder IL-15 ist.

4. Verfahren nach einem der Ansprüche 1 bis 3, wobei das Transposonverfahren ein PiggyBac-Verfahren ist.

5. Verfahren nach einem der Ansprüche 1 bis 4, wobei die genetisch veränderte Zellpopulation und die Zellpopulation von dem gleichen Individuum stammen.

6. Verfahren nach einem der Ansprüche 1 bis 5, wobei das CAR-Protein ein Protein ist, das eine Bindungsdomäne umfasst, die für den humanen Granulozyten-Makrophagen-Kolonie-stimulierenden Faktor (granulocyte-macrophage colony-stimulating factor; GM-CSF)-Rezeptor spezifisch ist, und die endogene Zellpopulation GM-CSF-Rezeptor exprimierende Zellen ist.

**Revendications**

1. Procédé de production de cellules de mammifère génétiquement modifiées, comprenant les étapes consistant à :

   a) introduire un polynucléotide codant pour une protéine de récepteur d'antigène chimérique (CAR) dans une population de cellules comprenant des lymphocytes T dérivés d'un mammifère par une méthode de transposon pour obtenir une population de cellules génétiquement modifiées ;
   b) fournir une population de cellules dérivées de PBMC dérivées du mammifère exprimant une protéine qui se lie au CAR, dans lequel ladite population de cellules est cultivée en présence d'une ou plusieurs cytokines, dans lequel ladite cytokine est GM-CSF et/ou IL-4 et dans lequel lesdites PBMC dérivées sont des PBMC utilisées sans traitement d'inactivation ; et
   c) réaliser un co-culture de la population de cellules génétiquement modifiées de l'étape a) et de la population de cellules de l'étape b).

2. Procédé selon la revendication 1, dans lequel l'étape c) est réalisée en présence d'une ou plusieurs cytokines.

3. Procédé selon la revendication 2, dans lequel la cytokine à l'étape c) est IL-7 et/ou IL-15.

4. Procédé selon l'une quelconque des revendications 1 à 3, dans lequel la méthode de transposon est une méthode PiggyBac.

5. Procédé selon l'une quelconque des revendications 1 à 4, dans lequel la population de cellules génétiquement modifiées et la population de cellules sont dérivées du même individu.

6. Procédé selon l'une quelconque des revendications 1 à 5, dans lequel la protéine CAR est une protéine comprenant un domaine de liaison spécifique pour le récepteur du facteur de stimulation des colonies de granulocytes-macrophages humains (GM-CSF), et la population de cellules endogènes est des cellules exprimant le récepteur de GM-CSF.

# Fig. 1

# Fig. 2

# Fig. 3

# Fig. 4

Fig. 5

Fig. 6

Fig. 7

EP 3 872 087 B1

Fig. 8

## Fig. 9

EP 3 872 087 B1

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5827642 A
- WO 2017061615 A

### Non-patent literature cited in the description

- **PARTOW et al.** Blood. American Society of Hematology, 2014, vol. 1, 311
- *N. Engl. J. Med.,* 2017, vol. 377, 2531-2544
- *N. Engl. J. Med.,* 2018, vol. 378, 439-448
- *J Immunotherapy,* 2013, vol. 36 (1), 1-2
- *Current Opinion in Biotechnology,* 2018, vol. 53, 164-181
- *Cytotherapy,* 2014, vol. 16, 1257-1269
- *Lancet,* 2015, vol. 385, 517-528
- *J Immunol Methods,* 01 April 2003, vol. 275 (1-2), 251-5
- *Human Gene Therapy,* 2010, vol. 21, 427-437
- *Molecular Therapy: Methods & Clinical Development,* 2018, vol. 3, 131-140
- **YANG XU et al.** *Blood,* 2014, vol. 123, 3750-3759
- *Oncoimmunology,* 2016, vol. 5 (12), e1253656
- **HUANG X ; GUO H et al.** *Mol Ther.,* 2008, vol. 16, 580-9
- **SINGH H ; MANURI PR et al.** *Cancer Res.,* 2008, vol. 68, 2961-71
- **DENIGER DC ; YU J et al.** *PLoS One.,* 2015, vol. 10, e0128151
- **SINGH H ; MOYES JS et al.** *Cancer Gene Ther.,* 2015, vol. 22, 95-100
- **HOU X ; DU Y et al.** *Cancer Biol Ther.,* 2015, vol. 16, 8-16
- **SINGH H ; HULS H et al.** *Immunol Rev.,* 2014, vol. 257, 181-90
- **MAITI SN ; HULS H et al.** *J Immunother.,* 2013, vol. 36, 112-23
- **NAKAZAWA Y ; HUYE LE et al.** *J Immunother.,* 2009, vol. 32, 826-36
- **GALVAN DL ; NAKAZAWA Y et al.** *J Immunother.,* 2009, vol. 32, 837-44
- **NAKAZAWA Y ; HUYE LE et al.** *Mol Ther.,* 2011, vol. 19, 2133-43
- **HUYE LE ; NAKAZAWA Y et al.** *Mol Ther.,* 2011, vol. 19, 2239-48
- **SAHA S ; NAKAZAWA Y et al.** *J Vis Exp.,* 2012, vol. 69, e4235
- **NAKAZAWA Y ; SAHA S et al.** *J Immunother.,* 2013, vol. 36, 3-10
- **SAITO S ; NAKAZAWA Y et al.** *Cytotherapy,* 2014, vol. 16, 1257-69
- **NAKAZAWA et al.** *Journal of Hematology & Oncology,* 2016, vol. 9, 27
- **ZOLA H. et al.** *Immunol Cell Biol.,* 1991, vol. 69 (6), 411-412
- **SAVOLDO et al.** *J Clin Invest,* 2011, vol. 121 (5), 1822-1826
- **HUYE et al.** *Mol Ther,* 2011, vol. 19 (12), 2239-2248